# EUROPEAN PATENT APPLICATION

(11) **EP 1 547 665 A1**
(43) Date of publication of application: **29.06.2005**
(21) Application number: 04425223.7
(22) Date of filing: 30.03.2004
(51) Int. Cl.: B01D 53/04, F25D 17/04, A61L 9/12, A61L 9/16, B01D 46/42

(54) **A bactericidal filter for gases in compartments of household refrigerators**

(30) Priority: 22.12.2003 IT RN20030045
(71) Applicant: Pellegrini, Alfredo, 60044 Fabriano (Ancona) (IT)
(72) Inventor: Pellegrini, Alfredo, 60044 Fabriano (Ancona) (IT)
(74) Representative: Lanzoni, Luciano

(57) **Abstract**

A filter (1) for gases contained inside a compartment (40) of a household refrigerator ( 41) comprises a substance, preferably a consumable gel (35), which includes essence oils that can be vaporised inside the refrigerator compartment (40) and have bactericidal effect on the gases contained therein.

## Description

The present invention relates to the technical sector of electrical appliance ― and in particular of refrigerators ― and it specifically concerns a filter incorporating bactericidal means.

In household refrigerator, there is a known problem with spontaneous bacterial proliferation within the compartment where the foods are preserved; this proliferation should be inhibited or otherwise keep in check for obvious hygienic reasons.

To solve this problem, various solution attempts are currently ongoing. A first technique being studied entails the use, in refrigerators, of electrical filters with bactericidal capabilities. Such a solution, a side from the complexity of construction and the cost it entails, has the fundamental drawback of not being generally usable, since it can be practically used with advantage only in the particular category of so-called "no-frost" refrigerators, which notoriously entail a mechanical ventilation of the air contained in the refrigerator compartment.

Another technique, based on a completely different approach, entails adding to the plastics, used in the construction of some internal parts of the refrigerator, bactericidal substances which are incorporated in the plastics during the moulding of the artefacts related thereto. Such an approach entails the advantage of avoiding the formation of bacterial charges and/or of moulds on the condensation formed in direct contact with the plastic materials, but does not exclude the ability of such undesired substances to proliferate undisturbed in other areas of the refrigerator cell or on parts made with different materials. It should also be stressed that such a solution is destined to have a bactericidal power that decreases progressively over time and that therefore diminishes its effectiveness during the working life of the refrigerator.

The object of the present invention is to overcome the aforesaid problems by means of a solution that is applicable to the generality of refrigerators, that is economical and that is able to involve the totality of the compartment for the preservation of the foods, irrespective of the nature of the constructive materials used, and which is also able to allow to restore bactericidal effectiveness when it is reduced or exhausted.

In accordance with the invention, said object is achieved by a filter for gaseous substances contained within a compartment of a household refrigerator, comprising activated charcoal and a substance, in particular a gel which is consumable in parallel with the progressive loss of filtering efficiency of the charcoals, and it is such that it also includes essence oils, able to be vaporised inside the refrigerator compartment and having bactericidal effect.

The technical features of the invention, according to the aforesaid objects, can be clearly noted from the content of the appended claims and its advantages shall be made more readily apparent in the detailed description that follows, made with reference to the accompanying drawings, which represent an embodiment provided purely by way of non limiting indication, in which:
- Figure 1 is a perspective exploded view illustrating a filter according to the invention;
- Figure 2 is an exemplifying diagram of the use of the filter according to the invention.

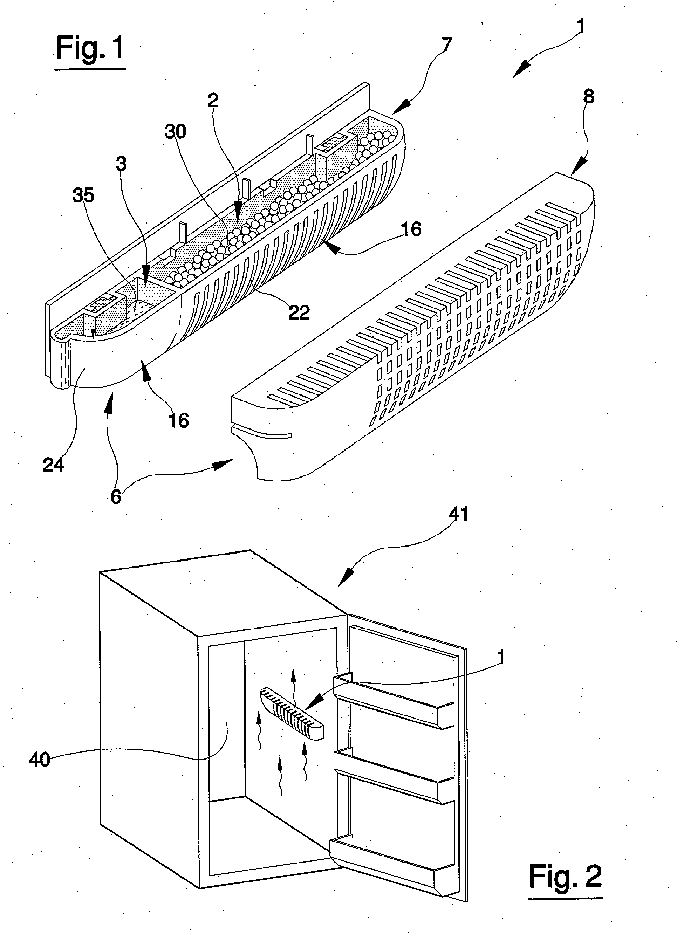

With reference to Figure 1 of the accompanying drawing, the number 1 globally designates a filter - the subject of a prior patent application by the same Applicant ― which comprises a container 6 constructed in two parts 7 and 8 which are structured in shell fashion and have mutually complementary shape so that they can be mutually coupled similarly to a body and to lid of a box structure.

The body 7 of the filter is delimited by a peripheral wall, globally designated by the reference number 16, which has a first grid-like part 22, provided with slits, and a second part 24 that is closed and contiguous to the previous one.

The first grid-like part 22 of the wall 16 delimits a first cup 2 internal to the body 7 of the filter 1 which contains a filtering substance preferably constituted by activated charcoals 30, in the solid state, granular, in a determined grain size.

The second part 24 of the lateral wall 16 of the body 7 of the filter 1 instead delimits a second cup 3 internal to the body 7 which contains a substance, preferably a gel 35, which by vaporising inside the refrigerator compartment 40 is progressively consumed serving as a visual sensor able to indicate to the observer the residual filtering efficiency still retained by the activated charcoals 35 after a certain period of use.

Preferably, to the gel 35 are added essence oils able to vaporise together with the gel 35 itself and to perform an intensive bactericidal action on the totality of the gases contained in the refrigerator compartment 40 destined to receive the foods.

There are numerous types of essence oils, usable as bactericidal substances; among them it is possible advantageously to use for example the natural substances belonging to the family of extracts from lavender, pine, cloves etc.

It should be noted that essence oils are able to carry out their effective bactericidal action indifferently, both in stationary air, and in air maintained in motion by ventilation systems of the type used in the technology known as "no-frost".

A filtering system according to the invention, in addition to the simplicity and economical construction of the filter 1 also has the advantage of being able to restore, through the replacement of the filter 1 itself, the optimal bactericidal potential once it is reduced or exhausted.

The above description refers for example to a filter 1 so structured as to have, as stated, two distinct cups 2 and 3 one for the charcoals 35 and one for the gel 30 incorporated in a single support body 7. However, this must be considered in wholly indicative and non limiting terms, since it is evident that the cup 3 for containing the bactericidal gel 35 can be wholly independent from the cup 2 of the charcoals 30 and even independent from the very presence of a charcoal filter which is located inside a refrigerator 41.
In the same way the essence oil can conceivably be inserted in an appropriate cup without any need to be mixed with the gel 35. The constructive solution of the cup will then cause the liquid (essence oil) to be contained with no possibility of being upset and at the same time with the capability to evaporate to go into circulation in the refrigerated space and to serve its bactericidal function.

The invention thus conceived is susceptible of evident industrial application; it can also be subject to numerous modifications and variants without thereby departing from the inventive concept. Moreover, all components can be replaced with technically equivalent elements.

## Claims

1. A filter for gases contained inside a compartment (40) of a household refrigerator (41), of the type comprising a consumable substance (35), **characterised in that** it includes essence oil, able to be vaporised inside the refrigerator compartment (40) and having bactericidal effect on the gases contained therein.

2. A filter, as claimed in claim 1, **characterised in that** said essence oils are contained in said consumable substance which is in the gel state (35).

3. A filter, as claimed in claim 1, **characterised in that** said essence oil are contained in a cup that is able to allow their evaporation.

4. A filter, as claimed in the previous claims, **characterised in that** said essence oils include substances included in the family of extracts from lavender, from pine, from cloves.

5. A filter, as claimed in the previous claims, **characterised in that** it comprises activated charcoals (30) whose residual efficiency is visually indicated by the residual content of said gel (35) in said filter (1).
